# EUROPEAN PATENT SPECIFICATION

(11) **EP 2 560 663 B1**
(45) Date of publication and mention of the grant of the patent: **22.06.2016**
(21) Application number: 11717157.9
(22) Date of filing: 22.04.2011
(51) Int. Cl.: A61K 33/42, A61K 9/08, A61P 9/10

(54) **METHODS AND COMPOSITIONS FOR REDUCING OR PREVENTING VASCULAR CALCIFICATION DURING PERITONEAL DIALYSIS THERAPY**
VERFAHREN UND ZUSAMMENSETZUNGEN ZUR SENKUNG ODER VORBEUGUNG VON GEFÄSSVERKALKUNGEN WÄHREND PERITONEALDIALYSEBEHANDLUNG
PROCEDES ET COMPOSITIONS POUR REDUIRE OU PREVENIR LA CALCIFICATION VASCULAIRE AU COURS D'UNE THÉRAPIE PAR DIALYSE PÉRITONÉALE

(30) Priority: 23.04.2010 US 327429 P
(43) Date of publication of application: 27.02.2013
(73) Proprietor: Baxter International Inc., Deerfield, IL 60015 (US); Baxter Healthcare S.A., 8152 Glattpark (Opfikon) (CH)
(72) Inventor: RISER, Bruce L., Kenosha Wisconsin 53140 (US); WHITE, Jeffrey A., Waukegan Illinois 60085 (US); DALTON, Christopher R., Mundelein Illinois 60060 (US)
(74) Representative: Potter Clarkson LLP
(86) International application number: PCT/US2011/033571
(87) International publication number: WO 2011/133855

(56) References cited:
- WO-A1-98/29434
- US-A1- 2005 142 212
- US-A1- 2007 148 258
- US-A1- 2009 098 215
- US-B1- 6 521 304
- US-B2- 7 267 986
- PEURAVUORI H ET AL: "Inorganic pyrophosphatase-labelled enzyme immunoassay for beta2-microglobulin - I Purification and catalytic properties", JOURNAL OF IMMUNOLOGICAL METHODS, ELSEVIER SCIENCE PUBLISHERS B.V.,AMSTERDAM, NL, vol. 204, no. 2, 26 May 1997 (1997-05-26), pages 161-167, XP004117437, ISSN: 0022-1759, DOI: 10.1016/S0022-1759(97)00042-2

## Description

### BACKGROUND

The present disclosure relates generally to medical treatments. More specifically, the present disclosure relates to methods and compositions for reducing, preventing or reducing the progression of vascular calcification in patients in conjunction with peritoneal dialysis therapy and the replacement of deficient pyrophosphate levels.

Due to disease, insult or other causes, a person's renal system can fail. In renal failure of any cause, there are several physiological derangements. The balance of water, minerals and the excretion of daily metabolic load are no longer possible in renal failure. During renal failure, toxic end products of nitrogen metabolism (e.g., urea, creatinine, uric acid and others) can accumulate in blood and tissues.

Kidney failure and reduced kidney function have been treated with dialysis. Dialysis removes waste, toxins and excess water from the body that would otherwise have been removed by normal functioning kidneys. Dialysis treatment for replacement of kidney functions is critical to many people because the treatment is life saving. One who has failed kidneys could not continue to live without replacing at least the filtration functions of the kidneys.

Past studies have shown that end stage renal disease ("ESRD") patients are deficient in pyrophosphate. Pyrophosphate may be instrumental in prevention of calcification of soft tissues and pyrophosphate deficiencies may be a risk factor in vascular calcification and calciphylaxis. The potential use of exogenously delivered pyrophosphate as a treatment or preventative, or indeed the verification of its role in preventing vascular calcification in vivo has not, however, been clearly demonstrated or actively pursued.

The stability of pyrophosphate is such that oral delivery of the molecule is not preferred because of low bioavailability through this administration route. Subcutaneous injection of pyrophosphate has been explored but development of skin necrosis makes this administration route not preferable.

Bisphosphonates, more chemically stable analogs of pyrophosphate, have been explored for the treatment of vascular calcification. Because their primary route of elimination is through the kidney and they are quite stable compounds, bisphosphonates can accumulate to toxic levels in patients with compromised or no kidney function. While a number of these analogs are currently used to treat osteoporosis, their use in end stage renal disease is contraindicated because patients cannot excrete the drug and unlike pyrophosphate they are not broken down by the ubiquitous circulating pyrophosphate degradative enzymes such as alkaline phosphatase. Their accumulation is thought to result in softening of bone thereby reducing their applicability.

US 2005/0142212 discloses a method for the prevention or treatment of phosphate or pyrophosphate depletion which involves the parenteral administration of pyrophosphate.

US 2009/0098215 discloses a dialysis solution comprising a therapeutically effective amount of pyrophosphate.

### SUMMARY

According to the present invention there is provided a dialysis solution comprising a therapeutically effective amount of pyrophosphate ranging between about 30 µM and about 400 µM for use in reducing or preventing the progression of vascular calcification in a patient by admnistration during peritoneal dialysis therapy.

The present disclosure relates to methods and compositions for reducing, preventing or reducing the progression of vascular calcification in patients. One method comprises administering to a patient during peritoneal dialysis therapy a dialysis solution including a therapeutically effective amount of pyrophosphate ranging between about 30 µM and about 400 µM. Formulations of dialysis solutions according to the dose ranges claimed in the present disclosure allow therapeutically effective amounts of pyrophosphate to be delivered to peritoneal dialysis therapy patients. The therapeutically effective amounts of pyrophosphate are sufficient to be maintained in the patient's body to reduce, prevent or reduce the progression of vascular calcification without adversely affecting the patient. The peritoneal dialysis therapy can be, for example, automated peritoneal dialysis, continuous ambulatory peritoneal dialysis or continuous flow peritoneal dialysis.

In another method, the pyrophosphate ranges between about 30 µM and about 300 µM in the dialysis solution. The dialysis solution can be in the form of a single solution, a concentrate that is subsequently diluted, or a multi-part dialysis product. The pyrophosphate can be pyrophosphoric acid, salt of pyrophosphate or a combination thereof. In one method, the pyrophosphate is tetra sodium pyrophosphate.

In another method, the dialysis solution includes one or more dialysis components including osmotic agents, buffers, electrolytes or a combination thereof. The osmotic agent can be glucose, glucose polymers, glucose polymer derivatives, cyclodextrins, modified starch, hydroxyethyl starch, polyols, fructose, amino acids, peptides, proteins, amino sugars, glycerol, N-acetyl glucosamine or a combination thereof. The buffer can be bicarbonate, lactate, pyruvate, acetate, citrate, tris, amino acids, peptides, an intermediate of the KREBS cycle or a combination thereof.

In another embodiment, the present disclosure provides a dialysis solution including a therapeutically effective amount of pyrophosphate ranging between about 30 µM and about 300 µM. The dialysis solution can be in the form of a single solution, a concentrate or a multi-part dialysis product. The pyrophosphate can be pyrophosphoric acid, salt of pyrophosphate or a combination thereof. In an embodiment, the pyrophosphate is tetra sodium pyrophosphate.

In an embodiment of the dialysis solution, the solution further includes one or more dialysis components such as osmotic agents, buffers, electrolytes or a combination thereof. The osmotic agent can be glucose, glucose polymers, glucose polymer derivatives, cyclodextrins, modified starch, hydroxyethyl starch, polyols, fructose, amino acids, peptides, proteins, amino sugars, glycerol, N-acetyl glucosamine or a combination thereof. The buffer can be bicarbonate, lactate, pyruvate, acetate, citrate, tris, amino acids, peptides, an intermediate of the KREBS cycle or a combination thereof.

In an embodiment of the dialysis solution, the dialysis solution can be in the form of at least two dialysis parts housed separately and the pyrophosphate is present with at least one of the dialysis parts and sterilized with said dialysis part.

In an alternative embodiment, the present disclosure provides a multi-part dialysis product including a first part having a concentrated pyrophosphate solution and/or a pyrophosphate powder, and a second part having a dialysis solution. The combination of the first part and the second part form a mixed solution having a therapeutically effective amount of pyrophosphate ranging between about 30 µM and about 400 µM.

Also described is a method of reducing, preventing or reducing the progression of vascular calcification in a patient. The method comprises administering to the patient during peritoneal dialysis therapy a concentrated pyrophosphate solution and/or a pyrophosphate powder that is diluted prior to or during the administration to provide the patient a therapeutically effective amount of pyrophosphate ranging between about 30 µM and about 400 µM. The concentrated pyrophosphate solution can be diluted with a separate dialysis solution prior to or during the administration.

An advantage of the present disclosure is to provide improved peritoneal dialysis therapies.

Another advantage of the present disclosure is to provide dialysis solutions including a therapeutically effective amount of pyrophosphate for reducing, preventing or reducing the progression of vascular calcification in patients.

Yet another advantage of the present disclosure is to provide improved methods of providing dialysis to patients.

Still another advantage of the present disclosure is to provide improved treatments for reducing, preventing or reducing the progression of vascular calcification in patients as a part of peritoneal dialysis therapies.

Another advantage of the present disclosure is replacing the "physiologically normal" level of pyrophosphate that was lost as a result of chronic kidney disease, kidney failure and/or dialysis, and required to not only prevent or treat vascular calcification, but also other conditions presently undefined, but requiring the normal physiological level.

Additional features and advantages are described herein, and will be apparent from, the following Detailed Description and the figures.

### DESCRIPTION OF THE FIGURES

FIG. 1 shows plasma pyrophosphate concentrations after intravenous administration of 4 ml/kg of 2.25 mM pyrophosphate solution (open squares) or intraperitoneal administration of 60 ml/kg of 0.150 µM pyrophosphate solution (open circles).
FIG. 2 shows in a quantitative manner the ApoE KO/CRF Model: Effect of Daily "Peritoneal Dialysis" with a pyrophosphate additive on Development of Calcification (Aortic Total Calcium).
FIGS. 3A and 3B show in a quantitative manner the ApoE/CRF Model: Effect of Daily "Peritoneal Dialysis" with a pyrophosphate additive on Development of Calcification (Aortic Valve Calcification).
FIG. 4 shows in a quantitative manner the ability of the doses tested in a pyrophosphate-containing solution to block the development of vascular calcification.

### DETAILED DESCRIPTION

The present disclosure relates to methods and compositions for reducing, preventing or reducing the progression of vascular calcification in patients. For example, the dialysis solutions in embodiments of the present disclosure are formulated to reduce, prevent or reduce the progression of vascular calcification due to pyrophosphate deficiencies or inadequacies in patients who have chronic renal disease, renal failure and/or are undergoing peritoneal dialysis therapies. The pyrophosphate is provided in a therapeutically effective amount in the dialysis solution so that it will remain at an effective level within the patient and will not adversely affect the health of the patient.

One method of reducing, preventing or reducing the progression of vascular calcification in a patient comprises administering to the patient during peritoneal dialysis therapy a dialysis solution including a therapeutically effective amount of pyrophosphate ranging between about 30 µM and about 400 µM. The patient may have, or be prone to, vascular calcification or have a pyrophosphate deficiency. Formulations of dialysis solutions, according to the dose ranges claimed in the present disclosure, allow therapeutic amounts of pyrophosphate to be delivered to peritoneal dialysis patients.

In another embodiment, the present disclosure provides a dialysis solution including a therapeutically effective amount of pyrophosphate ranging between about 30 µM and about 400 µM. More specifically, the amount of the pyrophosphate can be about 30 µM, 35 µM, 40 µM, 45 µM, 50 µM, 55 µM, 60 µM, 65 µM, 70 µM, 75 µM, 80 µM, 85 µM, 90 µM, 95 µM, 100 µM, 110 µM, 120 µM, 130 µM, 140 µM, 150 µM, 160 µM, 170 µM, 180 µM, 190 µM, 200 µM, 225 µM, 250 µM, 275 µM, 300 µM, 325 µM, 350 µM, 375 µM, 400 µM and the like. It should be appreciated that any two amounts of the pyrophosphate recited herein can further represent end points in a therapeutically preferred range of the pyrophosphate. For example, the amounts of 40 µM and 150 µM can represent the individual amounts of the pyrophosphate as well as a preferred range of the pyrophosphate in the dialysis solution between about 40 µM and about 150 µM.

Pharmacokinetic experiments were designed to examine the possibility that, when delivered via a peritoneal dialysis solution, exogenous pyrophosphate might enter the circulation slowly rather than being quickly degraded in the peritoneum and provide for continued administration with the potential for daily elevation of plasma and soft tissue levels. Studies regarding administration via an intraperitoneal or intravenous route showed that the intravenous delivered dose was available in the circulating plasma in its nearly full amount; however, it rapidly disappeared with a half-life of 5-10 minutes. In contrast, the same overall amount administered by way of a peritoneal dialysis solution through an intraperitoneal route, appeared more slowly in the plasma, peaking at a lower level with about 40% being biologically available and exhibiting a plasma half life of over two hours. With intraperitoneal administration, measurable levels of pyrophosphate persisted over several hours. This suggests the possibility of a superior delivery by the intraperitoneal route for replacing and maintaining pyrophosphate with the methods or formulations described herein.

It has been surprisingly found that the low end of the therapeutically effective range of pyrophosphate described herein is higher than what the skilled artisan would have understood based on the previous literature. The lower limit of the therapeutic pyrophosphate range in accordance with embodiments of the present disclosure was derived from a combination of data collected from a series of studies. For example, using an animal model that uniquely demonstrates disease similar to that developing in chronic kidney disease and end stage kidney disease on dialysis, it was found that solutions containing 150 µM pyrophosphate produced a complete blockade of vascular calcification. A decline in effectiveness was observed when the pyrophosphate concentration was reduced to 30 µM pyrophosphate indicating that 30 µM pyrophosphate produces some therapeutic effect. The inventors concluded that the lower limit of the therapeutic pyrophosphate range is about 30 µM pyrophosphate as concentrations of 30 µM pyrophosphate and higher are therapeutically effective while concentrations below 30 µM will likely no longer be effective.

It has also been surprisingly found that the upper end of the therapeutically effective and safe range of pyrophosphate is much lower than what the skilled artisan would have understood based on the previous literature. This was determined from a combination of animal efficacy and dose-finding studies as well as animal toxicity studies. A maximum tolerable dose suitable for administration was determined. At dosage amounts higher than about 400 µM pyrophosphate, the patient may experience adverse health due to toxicological effects. Chronic toxicity studies showed that, whereas systemic toxicity did not occur until pyrophosphate was dosed at upper millimolar concentrations (as reported in the literature), a local effect was observed in dose finding studies at 600 µM pyrophosphate and above and that the no-observed-effect level ("NOEL") was 300 µM pyrophosphate. Based on the absence of observed toxic effects at 300 µM pyrophosphate and the presence of observed toxic effects at 600 µM pyrophosphate, the inventors concluded that the upper limit of the therapeutic range is about 400 µM pyrophosphate.

The methods and dialysis solutions of the present disclosure can be used to replace the "physiologically normal" level of pyrophosphate in a patient that was lost as a result of chronic kidney disease, kidney failure and/or dialysis. The physiologically normal level of pyrophosphate is required to not only prevent or treat vascular calcification, but also other conditions presently undefined, but requiring the normal physiological level.

The dialysis solutions in any embodiments of the present disclosure can be sterilized using any suitable sterilizing technique such as, for example, autoclave, steam, high pressure, ultra-violet, filtration or combination thereof. The dialysis solutions can also be sterilized before, during or after one or more dialysis components and one or more pyrophosphates are combined.

The pyrophosphates can be, for example, pyrophosphoric acid, salts of pyrophosphate or combinations thereof. Salts of pyrophosphates include sodium pyrophosphate, potassium pyrophosphate, calcium pyrophosphate, magnesium pyrophosphate, etc. In an embodiment, the pyrophosphate is tetra sodium pyrophosphate.

In another embodiment, the present disclosure provides a dialysis solution including a therapeutically effective amount of pyrophosphate and one or more dialysis components such as osmotic agents, buffers and electrolytes. The dialysis solutions can preferably contain the dialysis components in an amount to maintain the osmotic pressure of the solution greater than the physiological osmotic pressure (e.g., greater than about 285 mOsmol/kg).

The osmotic agent can be glucose, glucose polymers (e.g., maltodextrin, icodextrin), glucose polymer derivatives, cyclodextrins, modified starch, hydroxyethyl starch, polyols (e.g., xylitol), fructose, amino acids, peptides, proteins, amino sugars, glycerol, N-acetyl glucosamine or combination thereof. The buffer can include bicarbonate, lactate, pyruvate, acetate, citrate, tris (i.e., trishydroxymethylaminomethane), amino acids, peptides, an intermediate of the KREBS cycle or a combination thereof. The electrolytes can include sodium, potassium, magnesium, calcium, chloride and the like suitable for dialysis treatments.

The bicarbonate buffer can be an alkaline solution such that the bicarbonate can remain stable without the use of a gas barrier overpouch or the like. The pH of the bicarbonate solution part can be adjusted with any suitable type of ingredient, such as sodium hydroxide and/or the like. Illustrative examples of the bicarbonate solution of the present disclosure can be found in U.S. Patent No. 6,309,673, entitled BICARBONATE-BASED SOLUTION IN TWO PARTS FOR PERITONEAL DIALYSIS OR SUBSTITUTION IN CONTINUOUS RENAL REPLACEMENT THERAPY, issued on October 30, 2001.

A variety of different and suitable acidic and/or basic agents can be utilized to adjust the pH of the osmotic, buffer and/or electrolyte solutions or concentrates. The acids can include one or more physiologically acceptable acids, such as lactic acid, pyruvic acid, acetic acid, citric acid, hydrochloric acid and the like. The acids can be in an individual solution having a pH that ranges from about 5 or less, about 4 or less, about 3 or less, about 2 or less, about 1 or less, and any other suitable acidic pH. The use of an organic acid, such as lactic acid, alone or in combination with another suitable acid, such as a suitable inorganic acid including hydrochloric acid, another suitable organic acid (e.g. lactic acid/lactate, pyruvic acid/pyruvate, acetic acid/acetate, citric acid/citrate) and the like in the acid solution can make the solution more physiologically tolerable according to an embodiment.

In alternative embodiments, the dialysis solution/concentrate can be in the form of a single peritoneal dialysis solution or a multi-part dialysis product including two or more dialysis parts (e.g., individual solutions/concentrates that make up the final dialysis solution when mixed) with each dialysis part including one or more dialysis components. An amount of pyrophosphate can be added to the single peritoneal dialysis solution or one or more of the dialysis parts of the multi-part dialysis product and sterilized with the dialysis part. The two or more dialysis parts can be stored and sterilized separately, for example, in separate containers or a multi-chamber container. When mixed, the resulting dialysis solution has a therapeutically effective amount of pyrophosphate.

In an alternative embodiment, the present disclosure provides a multi-part dialysis product including a first part having at least one of a concentrated pyrophosphate solution or a pyrophosphate powder, and a second part having a peritoneal dialysis solution. In an embodiment, the multi-part dialysis product is a single container having two separate parts and breaking a barrier or a peelable seal between the two parts of the multi-part dialysis product can make a final mixed solution having a therapeutically effective amount of pyrophosphate ranging between about 30 µM and about 400 µM. The dialysis solution can include one or more dialysis components such as osmotic agents, buffers, electrolytes or a combination thereof.

In another embodiment, the first part having the concentrated pyrophosphate solution or the pyrophosphate powder can be kept in a separate container or cartridge apart from the second part having the peritoneal dialysis solution (e.g., stored in a second container) to be subsequently mixed with the peritoneal dialysis solution at the time of the dialysis therapy using any suitable mixing techniques such as, for example, an automated peritoneal dialysis cycler. In this regard, the combination of the first part and the second part are capable of forming a mixed solution comprising a therapeutically effective amount of pyrophosphate ranging between about 30 µM and about 400 µM that can be administered to the patient.

It should be appreciated that the individual dialysis parts of the multi-part dialysis solutions can be housed or contained in any suitable manner such that the dialysis solutions can be effectively prepared and administered. A variety of containers can be used to house the two or more dialysis parts, such as separate containers (e.g., vials and bags) that are connected by a suitable fluid communication mechanism. The two or more separate parts of a dialysis solution can be separately sterilized and stored in the containers. In an embodiment, the pyrophosphate can be added to at least one of the dialysis parts and sterilized with that dialysis part. The dialysis part not containing the pyrophosphate can also be sterilized.

In an embodiment, the dialysis parts can be stored separately, for example, in separate compartments or chambers of the same container (e.g., of a multi- or twin-chambered bag) and combined prior to or during dialysis treatment. An activation of a barrier such as, for example, a peel seal or frangible between the chambers can allow for mixing of the contents of both chambers. The container can be covered with a gas impermeable outer-container. Alternatively, the sterilized dialysis parts can be stored separately and be combined at any time to form a complete ready-to-use dialysis solution as previously discussed.

As previously discussed, a suitable family of compounds capable of serving as osmotic agents in dialysis solutions is that of glucose polymers or their derivatives, such as icodextrin, maltodextrins, hydroxyethyl starch and the like. While these compounds are suitable for use as osmotic agents, they can be sensitive to low and high pH, especially during sterilization and long-term storage. Glucose polymers, such as icodextrin, can be used in addition to or in place of glucose in peritoneal dialysis solutions. In general, icodextrin is a polymer of glucose derived from the hydrolysis of corn starch. It has a molecular weight of 12-20,000 Daltons. The majority of glucose molecules in icodextrin are linearly linked with α (1-4) glucosidic bonds (>90%) while a small fraction (<10%) is linked by α (1-6) bonds.

The sterilized dialysis solutions of the present disclosure can be used in a variety of suitable peritoneal dialysis therapies. For example, the dialysis solutions can be used during peritoneal dialysis therapies such as automated peritoneal dialysis, continuous ambulatory peritoneal dialysis, continuous flow peritoneal dialysis and the like.

It should be appreciated that the dialysis solutions of the present disclosure can include any other suitable components/ingredients for dialysis treatment in addition to those components described above. In an embodiment, the pH of the single (e.g., mixed) dialysis solutions can have a broad range, preferably between about 4 to about 9. In another embodiment, the pH of the (mixed) dialysis solutions can have a broad range, preferably between about 5 to about 8.

Also described is a method of reducing, preventing or reducing the progression of vascular calcification in a patient. The method comprises administering to the patient during peritoneal dialysis therapy at least one of a concentrated pyrophosphate solution or a pyrophosphate powder that is diluted prior to or during the administration to provide the patient a therapeutically effective amount of pyrophosphate ranging between about 30 µM and about 400 µM. The concentrated pyrophosphate solution can be diluted with a separate dialysis solution prior to or during the administration. The concentrated pyrophosphate solution can be diluted with the dialysis solution automatically or manually using a suitable dialysis machine.

The dialysis solutions of the present disclosure can be made by any suitable methods. In an embodiment, the method comprises providing two or more solution parts with at least one part including one or more dialysis components such as an osmotic agent, a buffer or an electrolyte and another part including at least one of a concentrated pyrophosphate solution or a pyrophosphate powder. Alternatively, pyrophosphate in a therapeutically effective range as discussed above can be added to one or more separate dialysis parts of a multi-part dialysis product and sterilized with the dialysis part. The dialysis parts are subsequently mixed to form the final dialysis solution.

The sterilization can be performed, for example, by autoclave, steam, high pressure, ultra-violet, filtration or combination thereof. The sterilizing can be performed at a temperature and a pH that does not result in significant breakdown of the pyrophosphate in the dialysis solution. For example, a suitable buffer can be used to maintain the pH at a level that minimizes pyrophosphate degradation. In an alternative embodiment, the method comprises preparing a single dialysis solution including one or more of an osmotic agent, an electrolyte and a buffer along with a therapeutically effective amount of pyrophosphate and sterilizing the dialysis solution.

### EXAMPLES

By way of example and not limitation, the following examples are illustrative of various embodiments of the present disclosure and further illustrate experimental testing conducted with dialysis solutions including pyrophosphates.

### EXAMPLE 1

### Pharmacokinetic/bioavailability studies

Fifty-four male Sprague Dawley rats approximately 250 g each were randomized to two groups, intravenous ("IV") administration and intraperitoneal ("IP") administration. Both groups received a pyrophosphate ("PPi") dose of 2.0 mg/kg. Group 1 was administered a 4 mL/kg dose of pH adjusted (7.4) saline solution containing PPi 2.25 mM and P-32 labeled PPi (50 µCi, specific activity 84.5 Ci/mmol) via infusion through a tail vein as a single bolus, followed by a 0.2 mL saline flush. Group 2 was administered a 60 mL/kg dose of a 0.15 mM solution of PPi and P-32 labeled PPi (50 µCi, specific activity 84.5 Ci/mmol) in PHYSIONEAL® 40 dialysis solution via a single intraperitoneal injection. Blood (IV) and blood (IP) and peritoneal fluid (IP) were collected at various time points through 8 hours post dosing.

Plasma and peritoneal fluid were analyzed by two methods: a liquid scintillation method for total radioactive amounts and an HPLC method with radioactive detection for separation of pyrophosphate from phosphate and other phosphate-containing compounds.

Pharmacokinetic parameters were obtained from plasma and peritoneal fluid concentrations using a noncompartmental model. Parameters included determination of the maximum concentration ("Cₘₐₓ"), time to reach Cₘₐₓ ("Tₘₐₓ"), plasma half-life ("t_{1/2}") and area under the concentration time curve from 0 to last measurable time point (AUC₀₋ₜ). Bioavailability ("F") of the IP dose in plasma was calculated using F% = (AUC_{IP}/dose_{IP})/(AUC_{IV}/dose_{IV}) X 100. Pharmacokinetic data is shown in Tables 1-2.

**Table 1: Pharmacokinetic parameters determined from mean plasma concentrations of pyrophosphate following intravenous or intraperitoneal administration.**

| **Parameter** | **Intravenous** | **Intraperitoneal** |
|---|---|---|
| Dose (µg/kg) | 2035.15 | 2002.10 |
| Cmax (µg/mL) | 7.50 | 0.248 |
| Tₘₐₓ (hr) | 0.05 | 0.44 |
| t_{1/2} (hr) | 0.12 | 2.19 |
| AUC₀₋ₜ (hr*µg/mL) | 1.40 | 0.529 |
| F% | NA | 38.34% |

**Table 2: Pharmacokinetics of intravenous and intraperitoneal administration of pyrophosphate as measured using radiolabeled pyrophosphate**

| | **Intravenous** | | **Intraperitoneal** | |
|---|---|---|---|---|
| **Time point** | **Mean PPi (µg/mL)** | **Standard Error (µg/mL)** | **Mean PPi (µg/mL)** | **Standard Error (µg/mL)** |
| **Immediate** | 7.497 | 1.765 | 0.000 | 0.000 |
| **5 min** | 4.062 | 1.248 | 0.104 | 0.034 |
| **10 min** | 0.951 | 0.080 | 0.226 | 0.021 |
| **15 min** | 0.842 | 0.157 | 0.213 | 0.097 |
| **20 min** | 0.482 | 0.010 | 0.197 | 0.066 |
| **25 min** | 0.231 | 0.043 | 0.248 | 0.052 |
| **30 min** | 0.150 | 0.032 | 0.143 | 0.124 |
| **40 min** | 0.080 | 0.015 | 0.184 | 0.019 |
| **50 min** | 0.043 | 0.009 | 0.118 | 0.009 |
| **1 hr** | 0.021 | 0.007 | 0.134 | 0.025 |
| **1.5 hr** | 0.027 | 0.025 | 0.120 | 0.054 |
| **2 hr** | 0.010 | 0.017 | 0.121 | 0.009 |
| **3 hr** | 0.000 | 0.000 | 0.105 | 0.050 |
| **4 hr** | 0.012 | 0.021 | 0.021 | 0.037 |
| **6 hr** | 0.030 | 0.026 | 0.046 | 0.040 |
| **8 hr** | 0.030 | 0.009 | 0.000 | 0.000 |

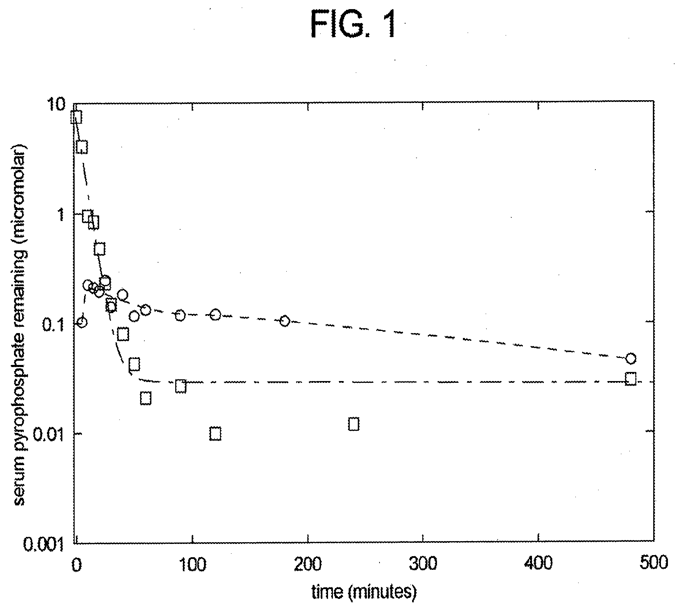

The data in Tables 1-2 and FIG. 1 provide evidence that intraperitoneal delivery of pyrophosphate results in a protracted delivery of pyrophosphate compared to that obtained by intravenous delivery of pyrophosphate. The protracted delivery is demonstrated by the longer half-life of PPi in plasma obtained by IP delivery (2.19 hours) as opposed to the shorter half-life of PPi in plasma (0.12 hours) obtained with IV delivery. The total delivered dose of pyrophosphate, given by AUC₀₋ₜ (hr*µg/mL), is lower in intraperitoneal administration than in intravenous administration. Equivalently, the bioavailability of pyrophosphate delivered intraperitoneally is less than 100% and is seen here to be 38%. The bioavailability of pyrophosphate delivered via intraperitoneal administration constitutes a novel finding. The pharmacokinetic data collected in these studies, and particularly the pyrophosphate bioavailability, demonstrate how one could successfully dose the drug and the limitations of such dosing.

### EXAMPLE 2

### Creation of vascular calcification as a model of human disease in the dialysis copulation

Homozygous apolipoprotein E knockout (apoE^{-/-}) mice were housed in polycarbonate cages in a pathogen-free, temperature-controlled (25 °C) room with a strict 12-hour light/dark cycle and with free access to lab chow and water. All procedures were in accordance with National Institutes of Health ("NIH") guidelines for the care and use of experimental animals (NIH publication No. 85-23).

Chronic renal failure ("CRF") was created in 8-wk-old female apoE^{-/-} mice, which were then randomly assigned to 4 groups as follows:
1) non-CRF - EKO (ApoE knockout) animals (Control group, 6 mice);
2) CRF / EKO animals treated with dialysis solution alone, with no PPi (CRF placebo group, 8 mice);
3) CRF / EKO animals treated by low dose PPi in dialysis solution (30 µM, which approximates to 0.21 mg PPi/kg body weight/day) (CRF PPi low dose group, 8 mice); and
4) CRF / EKO animals treated high dose of PPi in dialysis solution (150 µM, which approximates to 1.10 mg PPi/kg body weight/day) (CRF PPi high dose group, 8 mice).

A 2-step procedure was used to create CRF in the mice at 10 weeks of age. Briefly, at age of 8 weeks, cortical electrocauterisation was applied to the right kidney through a 2-cm flank incision and contralateral total nephrectomy was performed through a similar incision 2 weeks later. Other mice underwent a 2-step procedure of sham operations with decapsulation of both kidneys with a 14-day distance between the two operations. Blood samples were taken 2 weeks after nephrectomy, and intra-peritoneal catheters were implanted at this time.

Animals of the CRF group with a urea level > 20 mM (confirming renal impairment [normal mouse serum urea, ≤ 12 mM]) were subsequently randomized to the 3 CRF subgroups: two CRF subgroups were treated with PPi, at 2 different doses, (1 intraperitoneal injection/day for 6 days), and one CRF subgroup received the dialysis solution alone for a time period of 8 weeks. At the end of the study, the heart with the aortic root was then separated from ascending aorta. Cryosections of the aortic root tissue were used for quantification of vascular calcification and were used to assess atherosclerotic lesions at the site of the root. The thoracic part of the aorta was stored at -80 °C and used for quantification of calcium content.

Mice having high LDL will develop atherosclerosis. Those with the added partial nephrectomy will develop kidney impairment and were expected to produce marked vascular calcification of the heart and aorta, thus mimicking the disease seen in many dialysis patients. All groups were treated in a manner to mimic a peritoneal dialysis therapy, with a peritoneal dialysis solution either in the absence of PPi (sham and CRF placebo control groups) or containing two different doses of PPi (as outlined above).

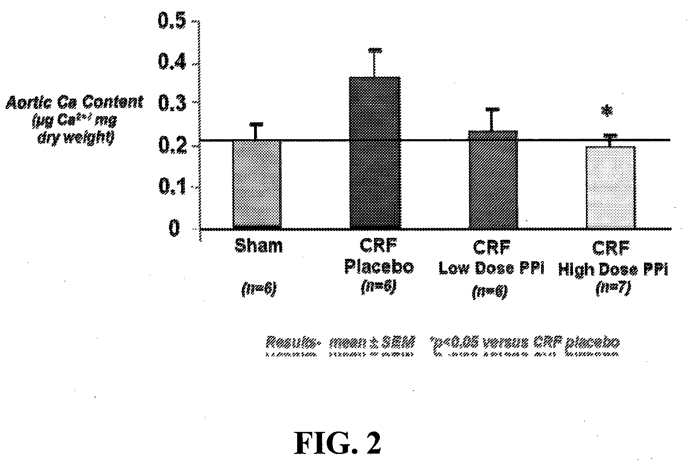

To determine the effect of PPi treatment via daily peritoneal delivery on vascular calcification ("VC"), total aortic calcium content was first measured. FIG. 2 shows that the creation of CRF induced a significant mean elevation (approximately 65%) of aortic calcification content compared to the sham group. Previous studies have shown that Apo-E KO mice have slightly elevated levels over the non-KO background strain (i.e., when neither are CRF). Treatment with the highest dose (150 µM) PPi completely blocked the effect of CRF on elevation of aortic calcium, producing mean values slightly below those in the sham group. A dose effect was observed, because the lower dose of PPi (30 µM) produced a moderate reduction, that was not statistically significant (FIG. 2).

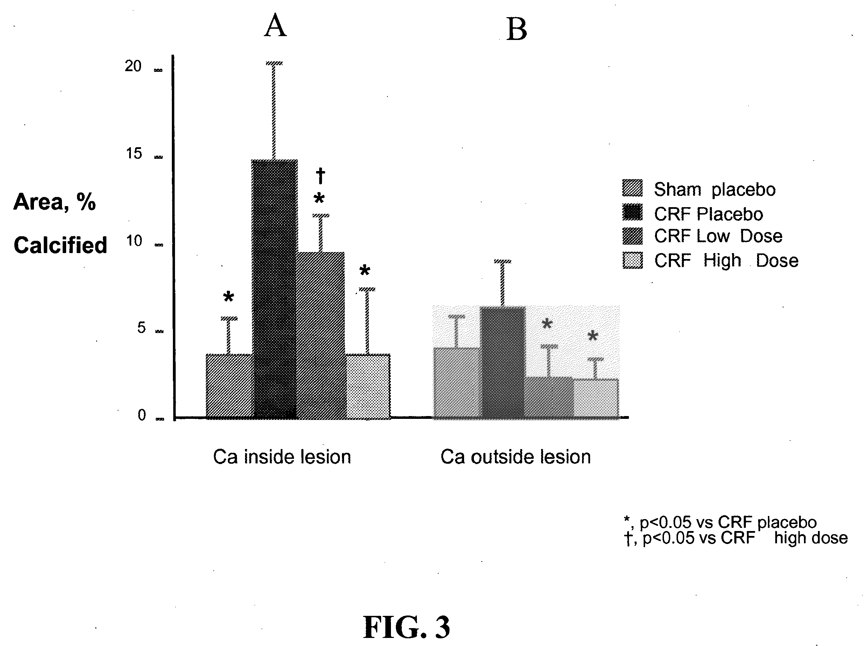

Next, a method was employed whereby morphological image processing algorithms were used for the semi-automated measurement of calcification from sections of aorta stained using von Kossa's silver nitrate procedure (FIGS. 3A and 3B). These were acquired at low magnification power on color images. The process was separated into two sequential phases: 1) segmentation to separate the calcification structures and demarcate the region of the atherosclerotic lesion within the tissue, and 2) the quantification. Calcified structures were measured inside and outside the lesion using a granulometric curve that allows the calculation of statistical parameters of size.

By using this method, quantification of calcification at the aortic root was determined and is shown in FIGS. 3A and 3B. The area of aortic root calcification measured inside the atherosclerotic lesion in the sham operated (non-CRF) group was observed to be greatly increased (approximately 5-fold) in animals with CRF (FIG. 3A). A strong dose-dependent blockade of this calcification was produced following treatment with PPi. The solution containing the high dose of PPi completely prevented the elevation due to CRF, whereas the solution with the low dose inhibited approximately 50% of the calcification inside the lesion. Examination of the calcification outside the lesion, assumed to be predominately medial calcification, was elevated greatly in the CRF group and was totally blocked by both doses of PPi. In both treated groups, the mean level of calcification appeared to be reduced below that of the sham placebo. Differences from the sham placebo were not statistically significant (FIG. 3B).

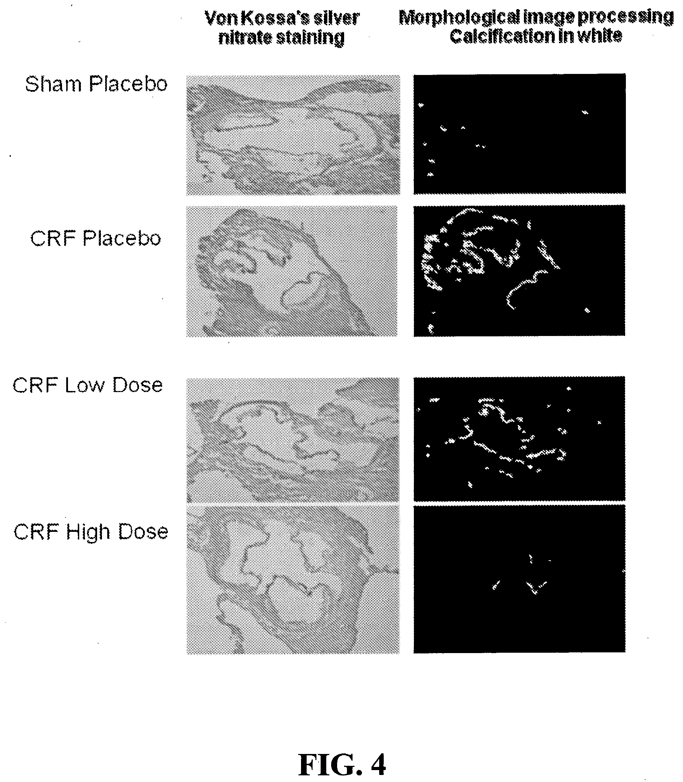

Photography of the typical staining is shown in a qualitative form in FIG. 4. Again, this demonstrates not only the ability of the treatment to block calcification but that the higher 150 µM dose totally blocks calcification whereas the lower 30 µM concentration produces a reduced but marked reduction in calcification.

The data in the APOe-KO/CRF mouse model of vascular calcification demonstrated that a concentration of 150 µM was sufficient to block the formation of all vascular calcification when administered daily in a peritoneal dialysis solution. The effect was reduced but still significant at a concentration of 30 µM. Consequently, the data from this efficacy study showed that the lower limit of the therapeutic range was about 30 µM pyrophosphate as concentrations of 30 µM pyrophosphate and higher provided therapeutic effectiveness while concentrations below 30 µM pyrophosphate would likely no longer be effective.

### EXAMPLE 3

A maximum tolerated dose study was conducted according to the study design in Table 3. Solutions that were administered were made up of a dextrose concentrate or a modified dextrose concentrate and a buffer concentrate or a modified buffer concentrate mixed in a 3:1 ratio (dextrose:buffer). The composition of the concentrates is shown in Tables 4-7. The PPi was included in the buffer or modified buffer solutions shown in Tables 5 and 7. Each test or control article was administered intraperitoneally once daily for 7 consecutive days to each of five different female rats at volumes of 40 mL/kg via a butterfly needle as a bolus injection. Necropsy was performed one day after the last dose.

Tissue samples of the diaphragms from all rats were trimmed, processed, embedded in paraffin, and sectioned. Hematoxylin and eosin stained slides were prepared and examined by light microscopy. Microscopic observations were subjectively graded based on the relative severity of the change: Grade 1 = minimal, Grade 2 = mild, Grade 3 = moderate, grade 4 = marked. Treatment related histopathologic changes in sections of diaphragm were limited to chronic inflammation of the peritoneal surface in rats given ≥ 600 µM pyrophosphate formulations.

Table 8 summarizes clinical observations following intraperitoneal administration of disodium pyrophosphate to the rats for seven days. In rats given 600 µM concentration, the diaphragmatic inflammation was graded mild (Grade 2) and was characterized by subserosal areas, rich in fibroblasts, with modest numbers of mononuclear inflammatory cells involving approximately 5-25% of the thickness of the section. At the 900 µM concentration, the reaction was mild in 1 rat and moderate in 4 of 5 rats and involved up to 50% of the thickness of the section. The reaction included small numbers of eosinophils and mast cells but otherwise was qualitatively similar to the reaction at 600 µM. The remaining histopathologic observation in the diaphragms from control and treated rats were considered nonspecific inflammatory or degenerative change not related to the administration of control or test articles.

**Table 3**

| **Description** | **Contents** | | |
|---|---|---|---|
| | **Bottle (A)** | **Bottle (B)** | **Final Solution PPi concentration** |
| Buffer Control | 75 mls Dextrose concentrate | 25 mls Buffer Concentrate | 0 |
| Test article 1 | 75 mls Dextrose concentrate | 25 mls Buffer Concentrate | 150 µM |
| Test Article 2 | 75 mls Dextrose concentrate | 25 mls Buffer Concentrate | 300 µM |
| Modified Buffer control | 75 mls Modified Dextrose concentrate | 25 mls Modified Buffer Concentrate | 0 |
| Test Article 3 | 75 mls Modified Dextrose concentrate | 25 mls Modified Buffer Concentrate | 600 µM |
| Test Article 4 | 75 mls Modified Dextrose concentrate | 25 mls Modified Buffer Concentrate | 900 µM |

**Table 4: Dextrose concentrate**

| **Component** | **MW (g/mol)** | **Concentration (mM)** | **Target Amount (per L)** |
|---|---|---|---|
| Dextrose, anhydrous | 180.2 | n/a | 51.53 g |
| Calcium chloride, dihvdrate | 147 | 1.67 | 0.245 g |
| Magnesium chloride, hexahydrate | 203.3 | 0.334 | 0.068 g |
| Sodium chloride | 58.44 | 114 | 6.66 g |
| Hydrochloric acid, 1N | n/a | n/a | 8.91 ml |
| Solution pH | n/a | n/a | pH 2.1 |
| Deionized water | To 1000 ml | | |

| | | | |
|---|---|---|---|
| n/a: not applicable | | | |

**Table 5: Buffer Concentrate**

| **Component** | **MW (g/mol)** | **Concentration (mM)** | **Target Amount (per L)** | **PPi Conc in Final mixed solution (µM)** |
|---|---|---|---|---|
| Sodium lactate | 112.06 | 60 | 6.72 g | 0 |
| Sodium bicarbonate | 84.01 | 111 | 9.33 g | |
| Sodium hydroxide, 1N | n/a | n/a | 16.1 mL | |
| Solution pH | n/a | n/a | pH 9.1 | |
| Di-sodium Pyrophosphate | 221.94 | 0.60 mM | 0.14 g | 150 |
| Di-sodium Pyrophosphate | 221.94 | 1.20 mM | 0.27 g | 300 |

| | | | | |
|---|---|---|---|---|
| n/a: not applicable | | | | |

**Table 6: Modified dextrose concentrate**

| **Component** | **MW (g/mol)** | **Concentration (mM)** | **Target Amount (per L)** |
|---|---|---|---|
| Dextrose, anhydrous | 180.2 | n/a | 51.53 g |
| Sodium chloride | 58.44 | 94.8 | 5.54 g |
| Hydrochloric acid, 1N | n/a | n/a | 13.3 mL |
| Solution pH | n/a | n/a | pH 2.1* |
| Deionized water | To 1000 ml | | |

| | | | |
|---|---|---|---|
| n/a: not applicable * adjust pH with additional HCl if needed | | | |

**Table 7: Modified buffer concentrate**

| **Component** | **MW (g/mol)** | **Concentration (mM)** | **Target Amount (per L)** | **PPi Conc. in Final mixed solution (µM)** |
|---|---|---|---|---|
| Sodium lactate | 112.06 | 60 | 6.72 g | 0 |
| Sodium bicarbonate | 84.01 | 111 | 9.33 g | |
| Sodium hydroxide, 1N | n/a | n/a | 41.67 mL | |
| Solution pH | n/a | n/a | pH 9.1* | |
| Di-sodium Pyrophosphate | 221.94 | 2.40 mM | 0.54 g | 600 |
| Di-sodium Pyrophosphate | 221.94 | 3.60 mM | 0.82 g | 900 |

| | | | | |
|---|---|---|---|---|
| n/a: not applicable * adjust pH with additional NaOH if needed | | | | |

**Table 8: Clinical observations following intraperitoneal administration of disodium pyrophosphate to rats for seven days**

| **Dosage (µM)** | **0** | **150** | **300** | **600** | **900** |
|---|---|---|---|---|---|
| Observations | 1 animal had mild diffuse subacute inflammation | No histopathological observations | 1 animal had minimal diffuse myofiber degeneration | All 5 animals had mild diffuse chronic inflammation of the peritoneum | All 5 animals had moderate diffuse chronic inflammation ofthe peritoneum. |
| | 1 had multifocal myofiber degeneration | No visible lesions | No visible lesions | No visible lesions | 4 of 5 animals had pale areas on diaphragm |
| | 1 had red cecum | | | | |

### Conclusions

The intraperitoneal administration of peritoneal dialysis solutions containing disodium pyrophosphate at concentrations of 150, 300, 600, or 900 µM to rats for 7 consecutive days at 40 mL/kg/day produced chronic inflammation of the peritoneal surface of the diaphragm at ≥ 600 µM pyrophosphate concentrations. Under the conditions of this study, the no-observed-effect level for the intraperitoneal administration of peritoneal dialysis solutions containing disodium pyrophosphate for 7 consecutive days was 300 µM at 40 mL/kg/day.

Prior studies from others on systemic toxicity indicated that doses well up into the millimolar range would be safe. The previous studies confirmed this with IV dosing, but when dosing via peritoneal dialysis, it was surprisingly found that much lower doses (600 µM) could cause intraperitoneal irritation with inflammation when chronically infused indicating additional limitations at concentrations of 600 µM. Based on the absence of observed toxic effects at 300 µM pyrophosphate and the presence of observed toxic effects at 600 µM pyrophosphate, the inventors concluded that the upper limit of the therapeutic range is about 400 µM pyrophosphate.

It should be understood that various changes and modifications to the presently preferred embodiments described herein will be apparent to those skilled in the art.

## Claims

1. A dialysis solution comprising a therapeutically effective amount of pyrophosphate ranging between about 30 µM and about 400 µM for use in reducing or preventing the progression of vascular calcification in a patient by administration during peritoneal dialysis therapy.

2. The solution for use according to Claim 1, wherein the pyrophosphate ranges between about 30 µM and about 300 µM.

3. The solution for use according to Claim 1 which has been prepared from a concentrate.

4. The solution for use according to Claim 1, wherein the pyrophosphate is selected from the group consisting of pyrophosphoric acid, salt of pyrophosphate and combinations thereof.

5. The solution for use according to Claim 1, wherein the pyrophosphate is tetra sodium pyrophosphate.

6. The solution for use according to Claim 1, wherein the solution comprises a dialysis component selected from the group consisting of osmotic agents, buffers, electrolytes and combinations thereof.

7. The solution for use according to Claim 6, wherein the osmotic agent is selected from the group consisting of glucose, glucose polymers, glucose polymer derivatives, cyclodextrins, modified starch, hydroxyethyl starch, polyols, fructose, amino acids, peptides, proteins, amino sugars, glycerol, N-acetyl glucosamine and combinations thereof.

8. The solution for use according to Claim 6, wherein the buffer is selected from the group consisting of bicarbonate, lactate, pyruvate, acetate, citrate, tris, amino acids, peptides, an intermediate of the KREBS cycle and combinations thereof.

9. The solution for use according to Claim 1, wherein the peritoneal dialysis therapy is selected from the group consisting of automated peritoneal dialysis, continuous ambulatory peritoneal dialysis and continuous flow peritoneal dialysis.

10. The solution for use according to Claim 1 which has been prepared by mixing together:
a first part comprising at least one of a concentrated pyrophosphate solution or a pyrophosphate powder; and
a second part comprising a dialysis solution.

## Patentansprüche

1. Dialyselösung umfassend eine therapeutisch wirksame Menge von Pyrophosphat in einem Bereich zwischen etwa 30 µM und etwa 400 µM für die Verwendung beim Verringern oder Verhindern des Fortschreitens von Gefäßverkalkung bei einem Patienten durch Verabreichen während Peritonealdialysetherapie.

2. Lösung für die Verwendung nach Anspruch 1, wobei das Pyrophosphat in einem Bereich zwischen etwa 30 uM und etwa 300 µM liegt.

3. Lösung für die Verwendung nach Anspruch 1, welche aus einem Konzentrat hergestellt worden ist.

4. Lösung für die Verwendung nach Anspruch 1, wobei das Pyrophosphat aus der Gruppe ausgewählt ist, bestehend aus Pyrophosphorsäure, Salz von Pyrophosphat und Kombinationen davon.

5. Lösung für die Verwendung nach Anspruch 1, wobei das Pyrophosphat Tetranatriumpyrophosphat ist.

6. Lösung für die Verwendung nach Anspruch 1, wobei die Lösung eine Dialysekomponente umfasst, ausgewählt aus der Gruppe bestehend aus Osmosemitteln, Puffern, Elektrolyten und Kombinationen davon.

7. Lösung für die Verwendung nach Anspruch 6, wobei das Osmosemittel aus der Gruppe ausgewählt ist, bestehend aus Glucose, Glucosepolymeren, Glucosepolymerderivaten, Cyclodextrinen, modifizierter Stärke, Hydroxyethylstärke, Polyolen, Fructose, Aminosäuren, Peptiden, Proteinen, Aminozuckern, Glycerin, N-Acetylglucosamin und Kombinationen davon.

8. Lösung für die Verwendung nach Anspruch 6, wobei der Puffer aus der Gruppe ausgewählt ist, bestehend aus Bicarbonat, Lactat, Pyruvat, Acetat, Citrat, Tris, Aminosäuren, Peptiden, einem Zwischenprodukt des KREBS-Zyklus und Kombinationen davon.

9. Lösung für die Verwendung nach Anspruch 1, wobei die Peritonealdialysetherapie aus der Gruppe ausgewählt ist, bestehend aus automatisierter Peritonealdialyse, kontinuierlicher ambulanter Peritonealdialyse und kontinuierlicher Durchflussperitonealdialyse.

10. Lösung für die Verwendung nach Anspruch 1, welche hergestellt worden ist durch Zusammenmischen von:
einem ersten Teil, umfassend eine konzentrierte Pyrophosphatlösung und/oder ein Pyrophosphatpulver; und
einem zweiten Teil, umfassend eine Dialyselösung.

## Revendications

1. Solution de dialyse comprenant une quantité thérapeutiquement efficace de pyrophosphate comprise entre environ 30 µM et environ 400 µM destinée à être utilisée dans la réduction ou la prévention de l'évolution de la calcification vasculaire chez un patient par l'administration d'un traitement pendant la dialyse péritonéale.

2. Solution destinée à être utilisée selon la revendication 1, dans laquelle le pyrophosphate est compris entre environ 30 µM et environ 300 µM.

3. Solution destinée à être utilisée selon la revendication 1 qui a été préparée à partir d'un concentré.

4. Solution destinée à être utilisée selon la revendication 1, dans laquelle le pyrophosphate est choisi dans le groupe constitué par l'acide pyrophosphorique, le sel de pyrophosphate et les combinaisons de ceux-ci.

5. Solution destinée à être utilisée selon la revendication 1, dans laquelle le pyrophosphate est le pyrophosphate tétrasodique.

6. Solution destinée à être utilisée selon la revendication 1, laquelle solution comprend un composant de dialyse choisi dans le groupe constitué par les agents osmotiques, les tampons, les électrolytes et les combinaisons de ceux-ci.

7. Solution destinée à être utilisée selon la revendication 6, dans laquelle l'agent osmotique est choisi dans le groupe constitué par le glucose, les polymères de glucose, les dérivés des polymères de glucose, les cyclodextrines, l'amidon modifié, l'hydroxyéthylamidon, les polyols, le fructose, les acides aminés, les peptides, les protéines, les sucres aminés, le glycérol, la N-acétylglucosamine et les combinaisons de ceux-ci.

8. Solution destinée à être utilisée selon la revendication 6, dans laquelle le tampon est choisi dans le groupe constitué par le bicarbonate, le lactate, le pyruvate, l'acétate, le citrate, le tris, les acides aminés, les peptides, un intermédiaire du cycle de KREBS et les combinaisons de ceux-ci.

9. Solution destinée à être utilisée selon la revendication 1, dans laquelle le traitement par dialyse péritonéale est choisi dans le groupe constitué par la dialyse péritonéale automatisée, la dialyse péritonéale continue ambulatoire et la dialyse péritonéale à flux continu.

10. Solution destinée à être utilisée selon la revendication 1 qui a été préparée en mélangeant :
une première partie comprenant au moins l'une d'une solution concentrée de pyrophosphate ou d'une poudre de pyrophosphate ; et
une deuxième partie comprenant une solution de dialyse.
